(19) Europäisches Patentamt
European Patent Office
Office européen des brevets

(11) EP 2 145 634 A1

(12) **EUROPÄISCHE PATENTANMELDUNG**

(43) Veröffentlichungstag:
**20.01.2010 Patentblatt 2010/03**

(51) Int Cl.:
***A61L 24/04*** *(2006.01)* ***C08G 18/10*** *(2006.01)*

(21) Anmeldenummer: **08012901.8**

(22) Anmeldetag: **17.07.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Bayer MaterialScience AG**
**51368 Leverkusen (DE)**

(72) Erfinder: **Die Erfindernennung liegt noch nicht vor**

(54) **Medizinische Klebstoffe zur Stillung schwerwiegender Blutungen und Abdichtung von Leckagen**

(57) Die vorliegende Erfindung betrifft neuartige, schnell härtende Klebstoffe auf Basis hydrophiler Polyisocyanat-Prepolymere für den Einsatz in der Chirurgie zur Stillung schwerwiegender Blutungen (Hämostase) und Abdichtung von Leckagen.

EP 2 145 634 A1

**Beschreibung**

Die vorliegende Erfindung betrifft neuartige, schnell härtende Klebstoffe auf Basis hydrophiler Polyisocyanat-Prepolymere für den Einsatz in der Notfallversorgung zur Stillung schwerwiegender Blutungen und Abdichtung von Leckagen.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Voraussetzung für eine effiziente Klebung der Cyanacrylate sind trockene Untergründe. Bei starken Blutungen versagt der Klebstoff.

Als Alternative zu den Cyanacrylaten stehen biologische Klebstoffe wie zum Beispiel BioGlue®, eine Mischung aus Glutaraldehyd und Bovinem Serumalbumin, diverse kollagen- und gelatinebasierte Systeme (FloSeal®) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass diese nur bei kleineren Verletzungen auf nicht gespanntem Gewebe verwendet werden können. Kollagen- und Gelatinebasierte Systeme wie FloSeal® dienen aussschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot® oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Auf die mögliche Anwendung von Polyurethanprepolymeren als Hämostatikum wird in den Artikeln "Isocyanate -terminated urethane prepolymer as bioadhesive material: evaluation of bioadhesion and biocompatibility, in vitro and in vivo assays" (Journal of Biomaterials Science, Polymer Edition (2001), 12(7), 707-719) und "Development of a biodegradable bioadhesive containing urethane groups" (Journal of Materials Science: Materials in Medicine (2008), 19 (1), 111-120) hingewiesen.

Es wurde nun gefunden, dass sich Formulierungen aus speziellen hydrophilen Polyurethanprepolymeren und aminofunktionellen Härtern ausgezeichnet als Hämostatikum zur Blutstillung einsetzen lassen. Daneben weisen die Formulierungen eine vorteilhafte Klebeigenschaft auf, so dass neben der Blutstillung gleichzeitig eine Fixierung des durch die Formulierug gebildeten Films auf der verletzten Stelle erreicht wird. Ferner können hierdurch insbesondere bei größeren Verletzung Gewebestücke wieder aneinandergefügt und fixiert werden, was für den Wundheilungsprozess vorteilhaft ist.

Gegenstand der vorliegenden Erfindung ist daher die Verwendung von Formulierungen umfassend

A) isocyanatfunktionelle Prepolymere erhältlich aus

A1) aliphatischen Isocyanaten und

A2) Polyolen mit zahlenmittleren Molekulargewichten von ≥ 400 g/mol und mittleren OH-Funktionalitäten von 2 bis 6,

B) eine Härterkomponente umfassend

B1)aminofunktionelle Asparaginsäureester der allgemeinen Formel (I)

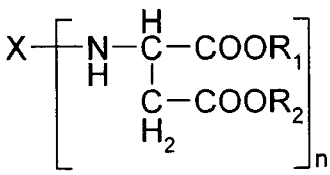

(I)

wobei

X ein n-wertiger organischer Rest ist, der durch Entfernung der primären Aminogruppen eines n-wertigen Amins erhalten wird,

$R_1$ , $R_2$ gleiche oder verschiedene organische Reste sind, die keinen Zerewitinoff-aktiven Wasserstoff aufweisen und

n eine ganze Zahl von mindestens 2 ist

und

B2)gegebenenfalls organische Füllstoffe, die eine nach DIN 53019 gemessenen Viskosität bei 23°C im Bereich von 10 bis 6000 mPas aufweisen

und

C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)

zur Stillung des Austritts von Blut (Hämostatikum) oder Gewebeflüssigkeiten oder dem Verschluss von Leckagen in Zellgeweben.

Ebenfalls ein Gegenstand der vorliegenden Erfindung ist die Verwendung vorgenannten Formulierungen zur Herstellung eines Mittels zur Stillung des Austritts von Blut (Hämostatikum) oder Gewebeflüssigkeiten oder dem Verschluss von Leckagen in Zellgeweben.

Ebenfalls ein Gegenstand der Erfindung ist ein Verfahren zur Stillung des Austritts von Blut (Hämostatikum) oder Gewebeflüssigkeiten oder dem Verschluss von Leckagen in Zellgeweben, bei dem die erfindungswesentlichen Formulierungen auf ein Zellgewebe aufgebracht und anschließend ausgehärtet werden.

Die erfindungswesentliche Stillung des Flüssigkeits- bzw. Blutaustritts bzw. der Verschluss von Leckagen in Zellgeweben kann sowohl in vivo als auch in vitro durchgeführt werden.

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Unter Gewebe oder Zellgewebe im Rahmen der vorliegenden Erfindung werden Zellverbände verstanden, die aus Zellen gleicher Gestalt und Leistung bestehen wie Deckgewebe (Haut), Epithelgewebe, Myocard, Binde- oder Stützgewebe, Muskeln, Nerven und Knorpel. Hierzu gehören unter anderem auch alle aus Zellverbänden aufgebaute Organe wie Leber, Niere, Lunge, Herz etc.

Die in A) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs- und Zusatzstoffen erhältlich.

In A1) können als Isocyanate beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisoeyanat (Nonan-triisocyanat), sowie Alkyl-2,6-diisocyanatohexanoate (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden in A1) Isocyanate der vorstehend genannten Art mit ausschließlich aliphatisch oder cycloaliphatisch gebundenen Isocyanatgruppen oder deren Mischungen eingesetzt.

Die in A1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in A1) Hexamethylendiisocyanat eingesetzt.

Zum Prepolymeraufbau können in A2) grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacryl-atpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

Die in A2) eingesetzten Polyole haben bevorzugt eine mittlere OH-Funktionalität von 3 bis 4

Die in A2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Bevorzugte Polyesterpolyol sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten $M_n$ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden die Verbindungen der Komponente A1) mit denen der Komponente A2) bei einem NCO/OH-Verhältnis von bevorzugt 4:1 bis 20:1, besonders bevorzugt 8:1 umgesetzt und anschließend der Anteil an nicht umgesetzten Verbindungen der Komponente A1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während oder nach der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

Bevorzugt sind in Formel (I):

$R_1$, $R_2$ gleiche oder verschiedene gegebenenfalls verzweigte oder cyclische organische Reste mit 1 bis 20, bevorzugt 1 bis 10 Kohlenstoffatomen, die keinen Zerewitinoff-aktiven Wasserstoff aufweisen,

n eine ganze Zahl von 2 bis 4 und

X ein n-wertiger organischer gegebenenfalls verzweigter oder cyclischer organischer Rest mit 2 bis 20, bevorzugt 5 bis 10 Kohlenstoffatomen, der durch Entfernung der primären Aminogruppen eines n-wertigen primären Amins erhalten wird.

Die Herstellung der aminofunktionellen Polyasparaginsäureester B1) erfolgt in bekannter Weise durch Umsetzung der entsprechenden primären mindestens difunktionellen Amine $X(NH_2)_n$ mit Malein- oder Fumarsäureestern der allgemeinen Formel

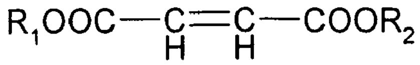

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine $X(NH_2)_n$ sind Ethylendiamin, 1,2-Diaminopropan, 1,4-Diaminobutan, 1,3-Diaminopentan, 1,5-Diaminopentan, 2-Methyl-1,5-Diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexyl-methan, 2,4,4'-Triamino-5-methyl-dicyclohexylmethan und Polyetheramine mit aliphatisch gebundenen primären Aminogruppen mit einem zahlenmittleren Molekulargewicht $M_n$ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

Bevorzugt sind $R_1$ und $R_2$ unabhängig voneinander $C_1$ bis $C_{10}$-Alkylreste, besonders bevorzugt Methyl oder Ethylreste.

In einer bevorzugten Ausführungsform der Erfindung sind $R_1 = R_2$ = Ethyl, wobei X auf 2-Methyl-1,5-Diaminopentan, als n-wertigem Amin basiert.

Bevorzugt ist n in Formel (I) für die Beschreibung der Wertigkeit des n-wertigen Amins eine ganze Zahl von 2 bis 6, besonders bevorzugt 2 bis 4.

Die Herstellung der aminofunktionellen Asparaginsäureester B1) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die in B2) eingesetzten organischen flüssigen Füllstoffe sind bevorzugt gemäß Zytotoxizitätsmessung nach ISO 10993 nicht zytotoxisch.

Beispielsweise können als organische Füllstoffe flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente B2) um hydroxy- oder aminofunktionelle, bevorzugt rein hydroxyfunktionelle Verbindungen. Bevorzugte rein hydroxyfunktionelle Verbindungen sind Polyether und/oder Polyesterpolyole, besonders bevorzugt Polyetherpolyole.

Die bevorzugten organischen Füllstoffe der Komponente B2) besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente B2) besitzen bevorzugt sich wiederholende von Ethylenoxid abgeleitete Einheiten.

Die Viskosität der organischen Füllstoffe der Komponente B2) beträgt bevorzugt 50 bis 4000 mPas bei 23°C gemessen nach DIN 53019.

In einer bevorzugten Ausführungsform der Erfindung werden als organische Füllstoffe der Komponente B2) Polyethylenglykole eingesetzt. Diese haben bevorzugt ein zahlenmittleres Molekulargewicht von 100 bis 1000 g/mol, besonders bevorzugt 200 bis 400 g/mol.

Das Gewichtsverhältnis von B1) zu B2) beträgt 1:0 bis 1:20, bevorzugt 1:0 bis 1:12.

Das Gewichtsverhältnis der Komponente B2 bezogen auf die Gesamtmenge der Mischung aus B1, B2 und A

liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in B1) und B2) eingesetzten Verbindungen auch die amino- oder hydroxyfunktionellen Umsetzungsprodukte isocyanatfunktioneller Prepolymere mit Asparaginsäureestern und/oder organischen Füllstoffen B2), sofern diese amino- oder hydroxyfunktionell sind, in einer separaten Vorreaktion herzustellen und dann als höhermolekulare Härterkomponente C) einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieses Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden.

Bei Bedarf kann eine der beiden Komponenten angefärbt werden.

Die erfindungswesentlichen Formulierungen werden durch Mischung des Prepolymers mit der Härterkomponente B) bzw. C) erhalten. In Komponente B) bzw. C) kann sich auch eine biologisch aktive Komponente D) befinden. Das Verhältnis von NCO-reaktiven NH-Gruppen zu freien NCO-Gruppen beträgt bevorzugt 1:1,5 bis 1:1, besonders bevorzugt 1:1.

Die erfindungswesentlichen Formulierungen besitzen unmittelbar nach Vermischung der Einzelkomponenten miteinander eine Scherviskosität bei 23°C von bevorzugt 1000 bis 10000 mPas, besonders bevorzugt 2000 bis 8000 mPas und ganz besonders bevorzugt 2500 bis 5000 mPas.

Die Geschwindigkeit bei 23°C bis eine vollständige Vernetzung und Aushärtung des Klebestoffs erreicht ist, beträgt typischerweise 30 s bis 10 min, bevorzugt 1 min bis 8 min.

Die erfindungswesentlichen Formulierungen können so wohl zur Stillung des Austritts von Blut- und Gewebeflüssigkeiten als auch zur Abdichtung von Leckagen im menschlichen oder tierischen Organismus sowie als Gewebeklebstoff angewendet werden, wobei die *in vivo* Anwendung beispielsweise zur Notfallbehandlung bei Polytraumata nach Unfällen oder Operationen bevorzugt ist.

**Beispiele:**

Sofern nicht abweichend angegeben beziehen sich alle Prozentangaben auf das Gewicht.

PEG = Polyethylenglykol

**Beispiel 1 (Prepolymere A)**

465 g HDI und 2.35 g Benzoylchlorid wurden in einem 1 l Vierhalskolben vorgelegt. Innerhalb von 2h wurden bei 80°C 931.8 g eines Polyethers mit einem Ethylenoxidgehalt von 71% und einem Propylenoxidgehalt von 29 % (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

**Beispiel 2 (Aspartat B)**

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Das Produkt wurde durch Destillation gereinigt.

**Beispiel 3 ( Anwendung der erfindungswesentlichen Formulierungen zur Stillung schwerwiegender Blutungen und Abdichtung von Leckagen)**

Die Applikation der erfindungswesentlichen Formulierungen erfolgte mittels eines käuflichen Zweikammerapplikators mit Statikmischer. Die eine Kammer enthielt dabei eine Mischung aus 0,45 g PEG 200 und 0,55 g Aspartat B. Die zweite Kammer enthielt 4 g des Prepolymers A. Durch Herabdrücken des Stempels kam es zu einer Durchmischung

der beiden Komponenten.

In vivo-Versuche zur Hämostase- Tiermodell Ratte

Der Versuch wurde bei einer 350 gr. schweren Wista Ratte durchgeführt. Die Narkoseeinleitung erfolgte mit Diethyläther und anschließend Ketamin/Xylazin intraperitoneal. Anschließend wurde die Trachea mit einem 14-Gauge Venenkatheter intubiert. Die Beatmung wurde mit einem LuftSauerstoff-Gemisch (FiO2=0,5) durchgeführt. Die Ratte wurde auf einer gewärmten Unterlage fixiert. Die chirurgische Präparation wurde aseptisch und unter lokaler Infiltration von Lidocain durchgeführt.

Das Abdomen wurde mittels anterioreren longitudinalen und transversen Bauchschnitts eröffnet, so dass ein weiter Zugang zur Leber und zur Milz erhalten wurde.

**Beispiel 3a - Diffuse Blutung**

Die Oberfläche der Leber und wurde mittels Sandpapier verletzt, so dass eine diffuse Blutung entstand. Die erfindungswesentliche Formulierung wurde auf die Oberfläche der Leber aufgebracht. Nach ca. 2 Minuten war der Film ausgehärtet und hatte die Blutung der Leberoberfläche gestillt.

**Beispiel 3b - Leberresektion**

Die Spitze des linken Leberlappens wurde entfernt. Dadurch entstand eine, quer durch das Lebergewebe verlaufende Schnittfläche von ca. $1cm^2$ mit starker Blutung. Die erfindungswesentliche Formulierung wurde aufgebracht. und stillte innerhalb von 2 Minuten die Blutung.

**Beispiel 3c- Lungenfunktion**

Der Brustkorb wurde mittels medianer Sternotomie eröffnet und mit einer rechts lateralen Thorakotomie erweitert. Am Mittellappen der rechten Lunge wurde die Spitze abgeschnitten, so dass eine ca. $1cm^2$ große Wundfläche entstand. Dabei entstanden eine starke venöse sowie eine starke arterielle Blutung. Zudem war ein mittelgroßer Bronchus durchtrennt worden, wodurch ein Luftleck entstand. Der Gewebeklebstoff wurde auf die Wundfläche der Lunge aufgebracht und stillte sofort die venöse sowie die arterielle Blutung. Bei dem Luftleck entstand eine große Luftblase im Klebstoff, die platzte und es bestand weiterhin eine Luftfistel. Nach ca. 1 Minute wurde erneut ein Tropfen des Klebstoffes auf das Luftleck aufgebracht und mit einem Plastikspatel festgedrückt. Dadurch war das Luftleck abgedichtet.

Nach insgesamt 3 Minuten war der Film ausgehärtet und hatte erfolgreich die Blutungen gestillt und das Luftleck abgedichtet.

**Beispiel 3d - Punktion der Aorta ascendens**

Die Aorta ascendens wurde freipräpariert und dargestellt. Die Aorta ascendens wurde mit einer 0.5mm dicken Nadel großzügig punktiert, so dass eine spritzende Blutung entstand. Die erfindungswesentliche Formulierung wurde auf die Blutung aufgebracht und mit einem Plastikspatel leicht auf das Loch gedrückt. Die Blutung kam innerhalb von 2 Minuten zum Stehen.

In vivo-Versuche zur Hämostase- Tiermodell Schwein

Der Versuch wurde an einem weiblichen 30 kg Hausschwein unter inhalativer Maskennarkose durchgeführt. Die Hautinzision erfolgte ventral des Musculus sternocleidomastoideus linksseitig. Die Aorta carotis wurde im Bereich des Bulbus freipräpariert. Die Aorta carotis stellt sich mit einem Durchmesser von ca. 5-6 mm dar.

**Beispiel 3e - Kleine arterielle Blutung**

Mit Hilfe eines Skalpells wurde die Arteria carotis im Bereich des Bulbus durch vorsichtige Präparation so eröffnet, dass es zu einer kleinen arteriell spritzenden Blutung kam. Nach kurzem Anspülen der Mischkanüle wurden ca. 4 ml der erfindungswesentlichen Formulierung auf die Blutungsquelle aufgebracht und mittels Kompression durch umliegendes Gewebe, v.a. durch den Musculus sternocleidomastoideus, komprimiert. Die Blutung stand nach ca. 1 ½ min. Das umliegende Gewebe war auf der Arteria carotis verklebt. Auf der Arteria carotis war distal der Inzisionsstelle ein Puls tastbar.

**Beispiel 3f - Schwerwiegende arterielle Blutung**

Mit Hilfe einer Gefäßschere wurde die Arteria carotis über die Hälfte der Circumferenz eröffnet. Es kam hierbei zu einer schweren spritzenden arteriellen Blutung. Es wurden 5 ml der erfindungswesentlichen Formulierung auf die Blutungsstelle aufgebracht und mit dem umliegenden Gewebe über ca. 2 min komprimiert. Die Blutung kam nach 2 Minuten zum Stillstand.

**Beispiel 3g - Venöse Blutung**

Die rechte Ohrvene wurde mit Hilfe eines Skalpells über ca. 10 mm längs eröffnet, wodurch es zu einer starken Blutung kam. Die erfindungswesentliche Formulierung wurde ohne Kompression aufgebracht. Nach ca. 1 Minute kam es zum Stillstand der Blutung.

**Patentansprüche**

**1.** Verwendung von Formulierungen umfassend

A) isocyanatfunktionelle Prepolymere erhältlich aus

A1) aliphatischen Isocyanaten und
A2) Polyolen mit zahlenmittleren Molekulargewichten von ≥ 400 g/mol und mittleren OH-Funktionalitäten von 2 bis 6,

B) eine Härterkomponente umfassend

B1) aminofunktionelle Asparaginsäureester der allgemeinen Formel (I)

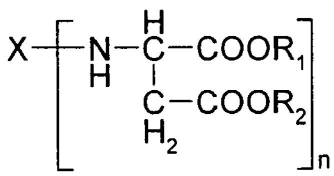
(I)

wobei

X ein n-wertiger organischer Rest ist, der durch Entfernung der primären Aminogruppen eines n-wertigen Amins erhalten wird,
$R_1$ , $R_2$ gleiche oder verschiedene organische Reste sind, die keinen Zerewitinoff-aktiven Wasserstoff aufweisen und
n eine ganze Zahl von mindestens 2 ist
und gegebenenfalls

B2) organische Füllstoffe, die eine nach DIN 53019 gemessenen Viskosität bei 23°C im Bereich von 10 bis 6000 mPas aufweisen

und
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)

zur Stillung des Austritts von Blut oder Gewebeflüssigkeiten oder dem Verschluss von Leckagen in Zellgeweben.

**2.** Verfahren zur Stillung des Austritts von Blut (Hämostatikum) oder Gewebeflüssigkeiten oder dem Verschluss von Leckagen in Zellgeweben, bei dem Formulierungen umfassend

A) isocyanatfunktionelle Prepolymere erhältlich aus

A1) aliphatischen Isocyanaten und
A2) Polyolen mit zahlenmittleren Molekulargewichten von ≥ 400 g/mol und mittleren OH-Funktionalitäten von 2 bis 6,

B) eine Härterkomponente umfassend

B1) aminofunktionelle Asparaginsäureester der allgemeinen Formel (I)

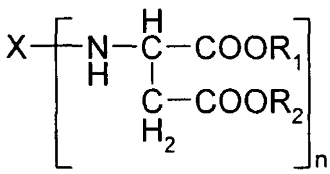

(I)

wobei

X ein n-wertiger organischer Rest ist, der durch Entfernung der primären Aminogruppen eines n-wertigen Amins erhalten wird,
$R_1$ , $R_2$ gleiche oder verschiedene organische Reste sind, die keinen Zerewitinoff-aktiven Wasserstoff aufweisen und
n eine ganze Zahl von mindestens 2 ist
und gegebenenfalls

B2) organische Füllstoffe, die eine nach DIN 53019 gemessenen Viskosität bei 23°C im Bereich von 10 bis 6000 mPas aufweisen

und
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)

auf ein Zellgewebe aufgebracht und anschließend ausgehärtet werden.

**3.** Verwendung von Formulierungen umfassend

A) isocyanatfunktionelle Prepolymere erhältlich aus

A1) aliphatischen Isocyanaten und
A2) Polyolen mit zahlenmittleren Molekulargewichten von ≥ 400 g/mol und mittleren OH-Funktionalitäten von 2 bis 6,

B) eine Härterkomponente umfassend

B1) aminofunktionelle Asparaginsäureester der allgemeinen Formel (I)

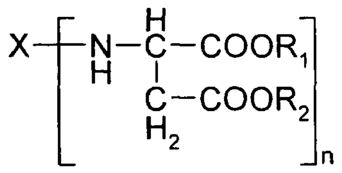

(I)

wobei

X ein n-wertiger organischer Rest ist, der durch Entfernung der primären Aminogruppen eines n-wertigen Amins erhalten wird,
$R_1$ , $R_2$ gleiche oder verschiedene organische Reste sind, die keinen Zerewitinoff-aktiven Wasserstoff aufweisen und
n eine ganze Zahl von mindestens 2 ist
und gegebenenfalls

B2) organische Füllstoffe, die eine nach DIN 53019 gemessenen Viskosität bei 23°C im Bereich von 10 bis 6000 mPas aufweisen

und
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)

zur Herstellung eines Mittels zur Stillung des Austritts von Blut oder Gewebeflüssigkeiten oder dem Verschluss von Leckagen in Zellgeweben.

**4.** Verwendung gemäß Anspruch 1 oder 3, **dadurch gekennzeichnet, dass** die in A2) eingesetzten Polyole zahlenmittlere Molekulargewichte von 4000 bis 8500 g/mol aufweisen.

**5.** Verwendung gemäß Anspruch 1, 3 oder 4, **dadurch gekennzeichnet, dass** in A2) Polyalkylenoxid-Polyether eingesetzt werden.

**6.** Verwendung gemäß Anspruch 5, **dadurch gekennzeichnet, dass** der Polyalkylenoxid-Polyether einen Gehalt an Ethylenoxid-basierten Einheiten von 60 bis 90 % bezogen auf die insgesamt enthaltene Mengen an Alkylenoxideinheiten aufweist.

**7.** Verwendung gemäß Anspruch 1 oder einem der Ansprüche 3 bis 6, **dadurch gekennzeichnet, dass** als organische Füllstoffe der Komponente B2) Polyetherpolyole eingesetzt werden.

**8.** Verwendung gemäß Anspruch 1 oder einem der Ansprüche 3 bis 7, **dadurch gekennzeichnet, dass** anstatt von der Härterkomponente B) ausschließlich zur Härtung der in A) eingesetzten Prepolymere die Umsetzungsprodukte gemäß C) eingesetzt wird.

**9.** Verwendung gemäß Anspruch 1 oder einem der Ansprüche 3 bis 8, **dadurch gekennzeichnet, dass** es sich um einen Gewebekleber für menschliches oder tierisches Gewebe handelt.

**10.** Verwendung gemäß Anspruch 1 oder einem der Ansprüche 3 bis 9, **dadurch gekennzeichnet, dass** es sich um eine in vivo oder eine in vitro Anwendung handelt.

Europäisches Patentamt
European Patent Office
Office européen des brevets

## EUROPÄISCHER RECHERCHENBERICHT

Nummer der Anmeldung
EP 08 01 2901

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 1 081 171 A (BAYER AG [US]) 7. März 2001 (2001-03-07) * Absatz [0006] * * Absatz [0009] * * Absatz [0041] * ----- | 1-10 | INV. A61L24/04 C08G18/10 |
| A | US 2004/067315 A1 (NIESTEN MEIKE [DE] ET AL) 8. April 2004 (2004-04-08) * Absatz [0006] - Absatz [0012] * * Absatz [0038] * ----- | 1-10 | |
| A | EP 1 719 530 A (TYCO HEALTHCARE [US]) 8. November 2006 (2006-11-08) * Absatz [0022] * * Absatz [0038] * * Absatz [0044] * * Absatz [0058] * * Absatz [0064] * ----- | 1-10 | |
| A | US 2005/129733 A1 (MILBOCKER MICHAEL T [US] ET AL) 16. Juni 2005 (2005-06-16) * Absatz [0021] * * Absatz [0023] * * Absatz [0035] - Absatz [0036] * ----- | 1-10 | RECHERCHIERTE SACHGEBIETE (IPC) A61L C08G |
| A | FERREIRA P ET AL: "Development of a biodegradable bioadhesive containing urethane groups" JOURNAL OF MATERIALS SCIENCE: MATERIALS IN MEDICINE, KLUWER ACADEMIC PUBLISHERS, BO, Bd. 19, Nr. 1, 21. Juni 2007 (2007-06-21), Seiten 111-120, XP019575568 ISSN: 1573-4838 * Seite 120 * ----- | 1-10 | |

Der vorliegende Recherchenbericht wurde für alle Patentansprüche erstellt

1

| Recherchenort | Abschlußdatum der Recherche | Prüfer |
|---|---|---|
| München | 5. November 2008 | Staber, Brigitte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 01 2901

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-2008

| Im Recherchenbericht angeführtes Patentdokument | | Datum der Veröffentlichung | Mitglied(er) der Patentfamilie | Datum der Veröffentlichung |
|---|---|---|---|---|
| EP 1081171 | A | 07-03-2001 | CA 2316531 A1 | 02-03-2001 |
| | | | DE 60016118 D1 | 30-12-2004 |
| | | | DE 60016118 T2 | 03-11-2005 |
| | | | ES 2233251 T3 | 16-06-2005 |
| | | | MX PA00008303 A | 06-08-2002 |
| | | | US 2002002300 A1 | 03-01-2002 |
| US 2004067315 | A1 | 08-04-2004 | AT 370976 T | 15-09-2007 |
| | | | AU 2003270263 A1 | 04-05-2004 |
| | | | DE 10246708 A1 | 15-04-2004 |
| | | | DK 1551894 T3 | 29-10-2007 |
| | | | WO 2004033517 A1 | 22-04-2004 |
| | | | EP 1551894 A1 | 13-07-2005 |
| | | | ES 2291667 T3 | 01-03-2008 |
| EP 1719530 | A | 08-11-2006 | AU 2006201642 A1 | 23-11-2006 |
| | | | CA 2543998 A1 | 05-11-2006 |
| | | | JP 2006312740 A | 16-11-2006 |
| | | | US 2006253094 A1 | 09-11-2006 |
| US 2005129733 | A1 | 16-06-2005 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 69311633 A


### In der Beschreibung aufgeführte Nicht-Patentliteratur


- Isocyanate -terminated urethane prepolymer as bioadhesive material: evaluation of bioadhesion and biocompatibility, in vitro and in vivo assays. Journal of Biomaterials Science, Polymer. 2001, vol. 12, 707-719
- Development of a biodegradable bioadhesive containing urethane groups. *Journal of Materials Science: Materials in Medicine,* 2008, vol. 19 (1), 111-120
- Römpp Chemie Lexikon. Georg Thieme Verlag